# EUROPEAN PATENT APPLICATION

(11) **EP 4 269 428 A1**
(43) Date of publication of application: **01.11.2023**
(21) Application number: 21911553.2
(22) Date of filing: 22.12.2021
(51) Int. Cl.: C07K 14/47, A61K 38/00

(54) **NOVEL PEPTIDE AND USE THEREOF**

(30) Priority: 22.12.2020 KR 20200180639
(71) Applicant: Medi & Gene, Seoul 02841 (KR); MEDI & GENE, Seoul 02841 (KR)
(72) Inventor: SHIN, Min Jeong, Seoul 06285 (KR); JEONG, In Hyeok, Goyang-si, Gyeonggi-do 10521 (KR); PARK, Chan, Uijeongbu-si, Gyeonggi-do 11627 (KR); LEE, Su Yeon, Seoul 01678 (KR)
(74) Representative: Vossius & Partner Patentanwälte Rechtsanwälte mbB
(86) International application number: PCT/KR2021/019658
(87) International publication number: WO 2022/139487

(57) **Abstract**

The present invention relates to an IF1 protein-derived peptide and a medicinal use thereof, in which the peptide suppresses appetite and inhibits lipid accumulation and differentiation of adipocytes in an obese animal model, and promotes insulin secretion and improves glucose tolerance in a diabetic animal model. Therefore, the present invention can be usefully used for anti-obesity and anti-diabetic purposes.

## Description

### [Technical Field]

The present invention relates to a novel peptide derived from a biological protein and an anti-obesity and anti-diabetic use of the same.

### [Background Art]

Pharmaceuticals used for the treatment and prevention of diseases are divided into pharmaceuticals including chemicals, and biopharmaceuticals. Biopharmaceuticals are mainly protein pharmaceuticals including hormones, antibodies, and the like, and additionally consist of genomes. Unlike pharmaceuticals that mainly include chemicals consisting of low-molecular-weight materials, biopharmaceuticals mostly consist of high-molecular-weight materials, so that various problems caused by their size occur. Typically, there are problems in terms of activity, such as deterioration in accessibility to a target substance and autoimmune response due to the large size, and industrial problems such as increases in cost and time caused by the increase in complexity involved in producing biopharmaceuticals as the size increases. This is a phenomenon that occurs because there are additional structural elements in addition to an active site, which has a major effect on the functions of proteins, and when the major active sites of proteins are identified, substantial improvements can be made in terms of various side effects and costs of protein pharmaceuticals because it is possible to reduce the size of protein pharmaceuticals while maintaining *in vivo* functions.

### [Disclosure]

### [Technical Problem]

Under the circumstances described above, the present inventors focused on the invention of peptides derived from IF1 protein in order to improve the function of ATPase inhibitory factor 1 (IF1) protein expressed *in vivo.* As a result, a novel IF1-derived peptide showing the effect of ameliorating metabolic disorders including obesity and glucose metabolism was found.

Therefore, an object of the present invention is to provide the novel peptide and a medical use of the same.

### [Technical Solution]

To achieve the object, an aspect of the present invention provides a peptide including an amino acid sequence represented by SEQ ID NO: 1 or 2 derived from a biological protein.

ATPase inhibitory factor 1 (IF 1) is known as a major protein that is expressed intracellularly and binds to ATPase present in the mitochondrial membrane to interfere with rotational movement, and consequently, affects intracellular energy regulation and mitochondrial homeostasis by interfering with ATP synthesis/degradation by the electron transport chain.

The present inventors discovered peptides of SEQ ID NOS: 1 and 2 that have anti-obesity and anti-diabetic activities by conducting research to find peptides with novel functionalities in the IF1 proteins (SEQ ID NOS: 3 and 4).

Another aspect of the present invention provides a pharmaceutical composition for preventing or treating obesity, including the peptide as an active ingredient.

As used herein, the term "prevention" refers to all actions that suppress the progression of a disease or delay the onset of the disease by administering the pharmaceutical composition according to the present invention.

As used herein, the term "treatment" refers to all actions that ameliorate or beneficially change symptoms of a disease by administering the pharmaceutical composition according to the present invention.

In order to confirm the physiological activity of a peptide derived from IF 1 protein (hereinafter referred to as a functional peptide), the present inventors co-administered a high fat diet and the functional peptide to mice (an obesity prevention experiment), or co-administered a high fat diet and the functional peptide to obesity-induced mice (an obesity treatment experiment).

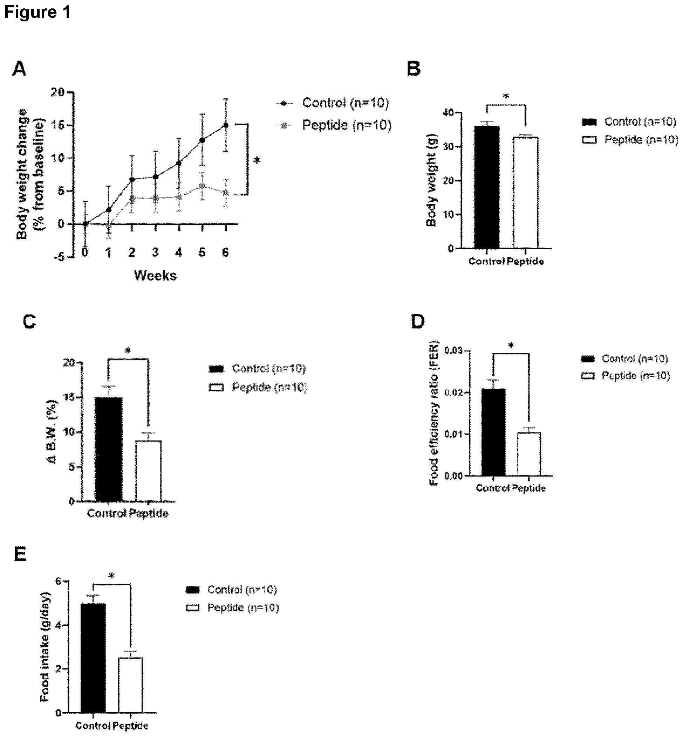

As a result of administration, the present inventors confirmed that the coadministration of the high fat diet and the functional peptide to mice suppressed body weight gain compared to the control (FIG. 1C) and confirmed that the administration of the high fat diet and the functional peptide to obesity-induced mice suppressed body weight gain, and actually decreased the body weight at the end of the experiment (FIGS. 1A and 1B).

Therefore, the composition may be usefully used for both prevention and treatment of obesity.

Meanwhile, the functional peptide suppresses lipid accumulation and differentiation of adipocytes by suppressing the expression of peroxisome proliferator-activated receptor gamma (PPARy), adiponectin, fatty acid-binding protein 4 (FABP4), CCAAT/enhancer-binding protein alpha (C/EBPα), and lipoprotein lipase (LPL) genes, which are indicators of adipose differentiation in adipocytes.

In addition, the functional peptide may suppress appetite by suppressing the expression of agouti-related protein (AgRP) and neuropeptide Y (NPY), which are peptides that promote appetite in hypothalamic cells.

Therefore, the composition for preventing or treating obesity may exhibit anti-obesity effects through suppression of adipocyte differentiation and appetite suppression.

Still another aspect of the present invention provides a pharmaceutical composition for preventing or treating diabetes or diabetic complications, including the functional peptide as an active ingredient.

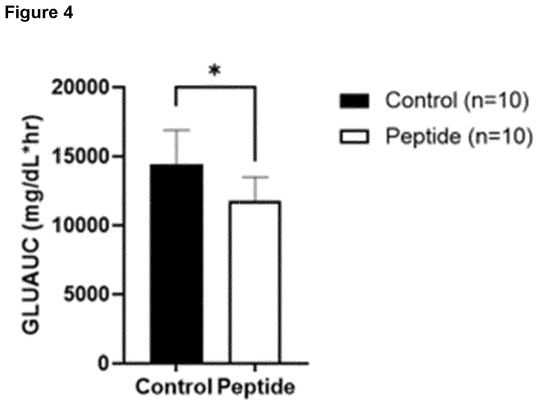

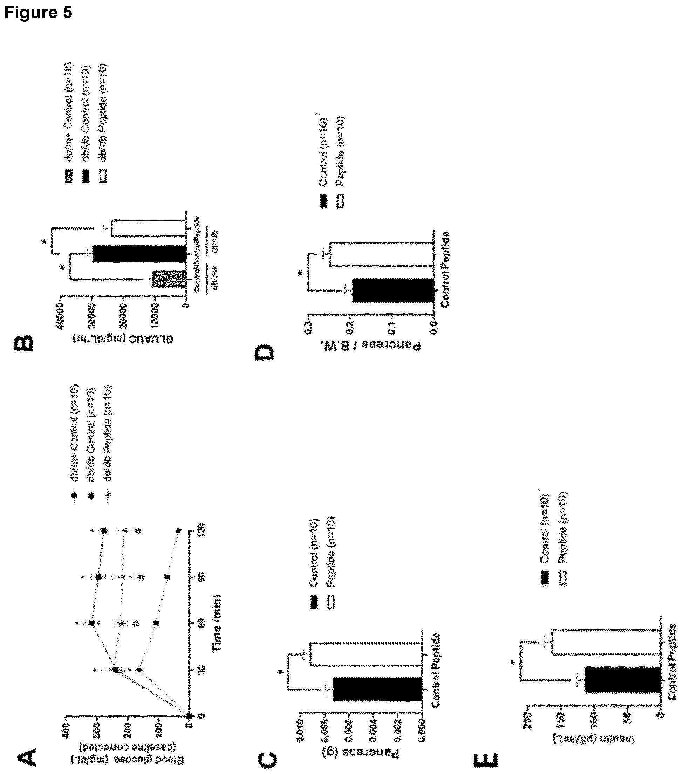

In order to additionally confirm the physiological activity of a functional peptide, the present inventors allowed obesity-induced mice and type 2 diabetic mice to fast and then injected a glucose solution into the mice to perform a glucose tolerance test. As a result, it was confirmed that by the administration of the functional peptide, blood glucose values were decreased (FIGS. 4, 5A, and 5B) and insulin secretions were increased (FIG. 5E).

Therefore, the composition for preventing or treating diabetes may exhibit anti-diabetic effects through improvement of glucose tolerance and promotion of insulin secretion.

Diabetic complications refer to complications such as myocardial infarction, cerebral stroke, retinopathy, and renal failure, which are caused by damage to blood vessel walls due to a long-term hyperglycemic state. Since diabetic complications may also be ameliorated when the hyperglycemic state is ameliorated, the functional peptide of the present invention may also be used for the use of preventing or treating diabetic complications.

Meanwhile, the pharmaceutical composition of the present invention may be prepared in a form additionally including a suitable carrier, excipient or diluent which is typically used in the preparation of pharmaceutical compositions, and the carrier may include a non-naturally occurring carrier.

Specifically, the pharmaceutical composition may be used by being formulated in the form of an oral formulation such as a powder, a granule, a tablet, a capsule, a suspension, an emulsion, a syrup, and an aerosol, an external preparation, a suppository, and a sterile injection solution, according to a typical method.

In the present invention, examples of a carrier, an excipient and a diluent which may be included in the pharmaceutical composition include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. When the pharmaceutical composition is prepared, the pharmaceutical composition is prepared using a diluent or excipient, such as a filler, an extender, a binder, a wetting agent, a disintegrant, and a surfactant, which are commonly used.

A solid formulation for oral administration includes a tablet, a pill, a powder, a granule, a capsule, and the like, and the solid formulation is prepared by mixing at least one excipient, for example, starch, calcium carbonate, sucrose or lactose, gelatin, and the like. Further, in addition to a simple excipient, lubricants such as magnesium stearate and talc are also used.

A liquid preparation for oral administration corresponds to a suspension, a liquid for internal use, an emulsion, a syrup, and the like, and the liquid formulation may include, in addition to water and liquid paraffin which are simple commonly used diluents, various excipients, for example, a wetting agent, a sweetener, an aromatic, a preservative, and the like. Examples of a preparation for parenteral administration include an aqueous sterile solution, a non-aqueous solvent, a suspension, an emulsion, a freeze-dried preparation, and a suppository. As the non-aqueous solvent and the suspending agent, it is possible to use propylene glycol, polyethylene glycol, a vegetable oil such as olive oil, an injectable ester such as ethyl oleate, and the like.

The pharmaceutical compositions may be in the form of a sterile injectable preparation as a sterile injectable aqueous or oily suspension. The suspension may be formulated according to techniques known in the art using a suitable dispersant or wetting agent (for example, Tween 80) and a suspending agent. The sterile injectable preparation may also be a sterile injectable solution or suspension in a nontoxic parenterally acceptable diluent or solvent (for example, a solution in 1,3-butanediol). Examples of a vehicle and a solvent that may be acceptably used include mannitol, water, Ringer's solution, and an isotonic sodium chloride solution. Furthermore, sterile, non-volatile oils are typically used as a solvent or suspending medium. Any mild or non-volatile oil, including synthetic mono- or diglycerides, may be used for this purpose. Natural oils in which fatty acids such as oleic acid and glyceride derivatives thereof are pharmaceutically acceptable (for example, olive oil or castor oil), particularly polyoxyethylated versions thereof, are also useful in injectable preparations.

Parenteral administration of the pharmaceutical composition according to the present invention is particularly useful when a target treatment is associated with sites or organs readily accessible by topical application. Examples of a carrier for topical administration of the compound of the present invention include, but are not limited to, mineral oil, liquid paraffin, white petrolatum, propylene glycol, polyoxyethylene, polyoxypropylene compounds, emulsifying wax and water.

The content of the active ingredient included in the pharmaceutical composition of the present invention is not particularly limited, but the active ingredient may be included in a content of 0.0001 to 50 wt%, more preferably 0.01 to 10 wt% based on the total weight of a final composition.

Yet another aspect of the present invention provides a method for treating obesity, the method including administering the pharmaceutical composition for preventing or treating obesity to an individual in need of treatment, and a method for treating diabetes or diabetic complications, the method including administering the pharmaceutical composition for preventing or treating diabetes or diabetic complications to an individual in need of treatment.

Since the above-described pharmaceutical compositions are used in the treatment method of the present invention, the description of the content common between the two will be omitted in order to avoid the excessive complexity of the present specification.

The method for administering the pharmaceutical composition may be oral or parenteral as described above, and the dosage of the pharmaceutical composition may be determined by a therapeutic expert.

### [Advantageous Effects]

The present invention relates to a biological protein-derived peptide and a medicinal use thereof, in which the peptide suppresses appetite and inhibits lipid accumulation and differentiation of adipocytes in an obese animal model, and promotes insulin secretion and improves glucose tolerance in a diabetic animal model. Therefore, the present invention can be usefully used for anti-obesity and anti-diabetic purposes.

### [Description of Drawings]

In FIG. 1, A shows the results of confirming changes in body weight while administering an IF1-derived peptide (hereinafter, described as a functional peptide) to a mouse model in which obesity was induced by a high fat diet for 6 weeks, B shows the results of confirming the final body weight after the administration for 6 weeks, and C to E show the results of confirming, after co-administering a high fat diet and a functional peptide to mice, the rate of the variations in total body weight during the administration period (C), the food intake efficiency during the administration period (D), and the daily food intake during the administration period (E): control: a control; and peptide: a functional peptide administration group (the same applies to the following drawings).

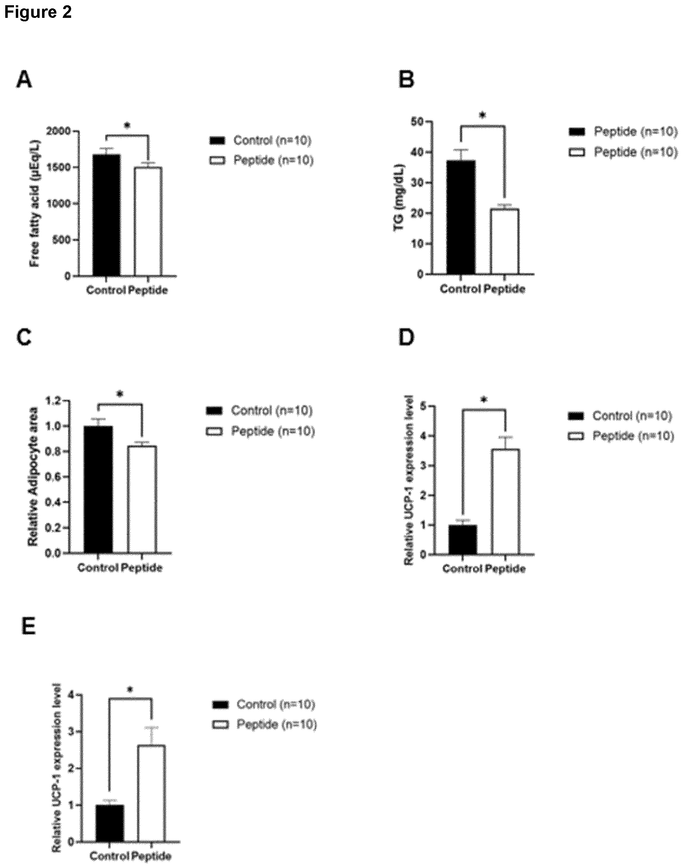

FIG. 2 shows the results of confirming the serum triglyceride level (A), serum free fatty acid level (B), area occupied by adipocytes in the adipose tissue (C) and changes in UCP-1 expression in adipose tissue (D), after co-administering a high fat diet and a functional peptide to mice for 30 days, and E shows the results of confirming changes in UCP-1 expression in adipose tissue after co-administering a high fat diet and a functional peptide to obesity-induced mice for 6 weeks.

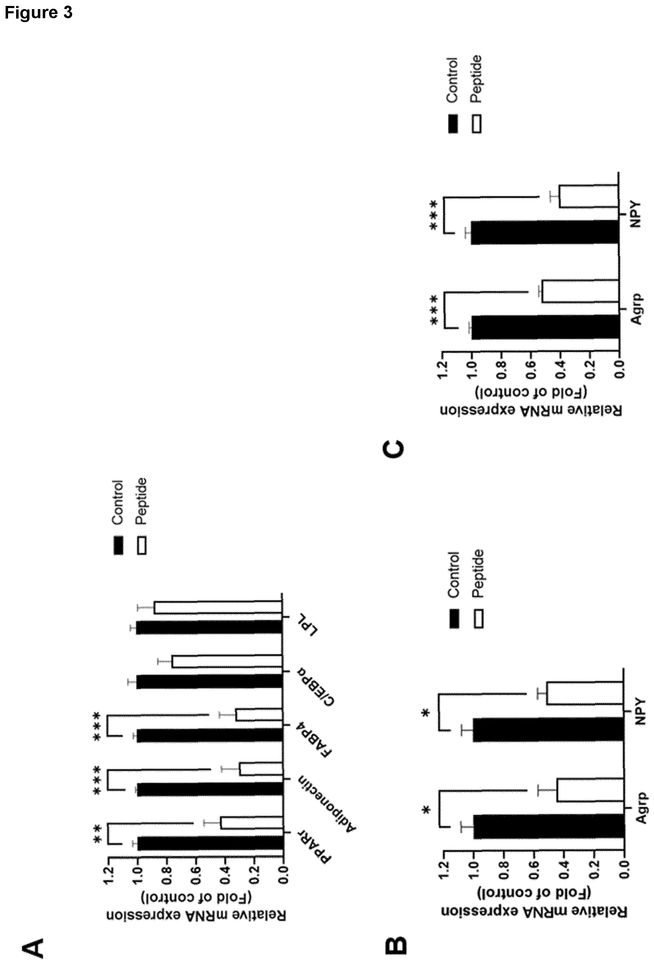

In FIG. 3, A shows the results of confirming changes in the expression of genes involved in adipocyte differentiation and growth after treatment with functional peptides during the differentiation process of adipocytes, and B and C shows the results of confirming changes in the expression of appetite-promoting genes after treating hypothalamic cells with mouse-derived and human-derived functional peptides, respectively.
FIG. 4 shows the results of confirming the variations in total blood glucose by allowing a mouse model with obesity induced by a high fat diet to fast, and then injecting a glucose solution into the mouse model.
In FIG. 5, A shows the results of confirming blood glucose values by allowing a type 2 diabetic mouse model to fast, and then injecting a glucose solution into the mouse model, B shows the results of quantifying the variations in total blood glucose, C shows the results of measuring the weight of the pancreas isolated from the mouse, D shows the results of comparing the result values of correcting the weight of the pancreas with the body weight of the mouse, and E shows the results of confirming the blood insulin concentration.

### [Modes of the Invention]

Hereinafter, one or more specific exemplary embodiments will be described in more detail through examples. However, these examples are provided only for exemplarily explaining the one or more specific exemplary embodiments, and the scope of the present invention is not limited to these examples.

### Example 1: Isolation and purification of IF1-derived peptides

IF1 protein and IF1-derived peptide (hereinafter referred to as a functional peptide; SEQ ID NO: 1 or 2) coding sequences were inserted into pGEX-4T-1 and pet28a vectors, respectively. BL21 (DE3), which is an *E. coli* strain for expression, was transformed with the vector to establish a cell model that stably expresses IF1 and the functional peptide. The corresponding *E. coli* cells were cultured, and then treated with 1 mM isopropyl β-D-1-thiogalactopyranoside (IPTG) to induce protein expression. Thereafter, a solution obtained through centrifugation and sonication processes was separated by affinity chromatography to separate IF1 and a functional peptide, and purified by adjusting the salt concentration with a PBS solution of pH 7.4 using Snakeskin dialysis tubing and increasing the purity. GST used as a control was produced using a pGEX-4T-1 vector instead of a pet28a vector.

The sequences of the IF1 protein and the functional peptide are shown in the following Table 1.

**[Table 1]**

| SEQ ID NO: | Sequence name | Sequence |
|---|---|---|
| 1 | Functional peptide (Mouse) | VSDSSDSMDTGAGSIREAGGAFGKREKAEEDRYFREKTK EQLAALRK |
| 2 | Functional peptide (Human) | GSDQSENVDRGAGSIREAGGAFGKREQAEEERYFRAQSR EQLAALKK |
| 3 | IF1 (Mouse) | VSDSSDSMDTGAGSIREAGGAFGKREKAEEDRYFREKTK EQLAALRKHHEDEIDHHSKEIERLQKQIERHKKKIQQLK NNH |
| 4 | IF1 (Human) | GSDQSENVDRGAGSIREAGGAFGKREQAEEERYFRAQSR EQLAALKKHHEEEIVHHKKEIERLQKEIERHKQKIKMLK HDD |

### Example 2: Anti-obesity effect analysis of functional peptides

### 2-1. Construction of obese mouse model

4-week-old C57BL/6J male mice were distributed by ORIENT BIO, fed with normal diet chow (ND) for 1 week, and subjected to an adaptation time. In this case, the temperature and humidity of the breeding space were maintained at all times at 18 to 24°C and at 50 to 60%, respectively, as breeding environments throughout the experimental period, and the mice were fed freely during both the adaptation period and the experimental period. After the adaptation period, the mice were divided into an obesity prevention experimental group and an obesity treatment experimental group, and bred according to each purpose. A normal diet and a high fat diet were prepared as shown in the following Table 2 and provided.

**[Table 2]**

| Component | Normal diet (g) | High fat diet (g) |
|---|---|---|
| Corn starch | 15 | 15 |
| Casein | 20 | 20 |
| Sucrose | 50 | 34 |
| Corn oil | 5 | 3 |
| Mineral mix | 3.5 | 3.5 |
| Vitamin mix | 1 | 1 |
| Cellulose | 5 | 5 |
| DL-methionine | 0.3 | 0.3 |
| Choline bitartrate | 0.2 | 0.2 |
| Lard | | 17 |
| Cholesterol | | 1 |
| BHT | 0.001 | 0.001 |
| Total | 100 (g) | 100 (g) |

- Obesity prevention experimental group: mice were divided into a control and an experimental group and each bred under the following conditions for 30 days.
   Control: High fat diet (HFD) + administration of PBS (n=10)
   Experimental group: High fat diet (HFD) + administration of functional peptide (n=10)
- Obesity treatment experimental group: after a 1-week adaptation period, mice were fed a high fat diet (see Table 1) for 8 weeks to induce obesity, divided into a control and an experimental group, and bred under the following conditions for 6 weeks.
   Control: High fat diet (HFD) + administration of PBS (n=10)
   Experimental group: High fat diet (HFD) + administration of functional peptide (n=10)

### 2-2. Anti-obesity effect of functional peptides

Under the experimental conditions of Example 2-1, PBS and a peptide were administered through intraperitoneal injection once daily during the experimental period. The corresponding conditions are as follows.
Control: intraperitoneal injection of PBS (volume corresponding to the following functional peptide solution)
Experimental group: intraperitoneal injection of functional peptide (5 mg/kg)

All mice were weighed during the experimental period to observe changes in body weight according to the intake of the high fat diet. As a result, it was confirmed that the functional peptide administration group in the obesity treatment experimental group showed an effect of suppressing body weight gain at the time point of one week after the administration of the peptide, and the suppression of body weight gain lasted until the end of the experiment (FIG. 1A). As a result of comparing the body weight at the end of the final experiment, obesity was rapidly induced in the control due to body weight gain caused by the high fat diet, but the functional peptide administration group rather showed a body weight loss effect (FIG. 1B).

In the obesity prevention experimental group, the functional peptide administration group also showed a low level of change in body weight during the entire experimental period (FIG. 1C), and an effective anti-obesity efficacy of the functional peptide was confirmed by confirming that the diet efficiency (the ratio of body weight gain divided by food intake) (FIG. 1D) and the daily food intake decreased in the functional peptide administration group (FIG. 1E).

After the end of the experiment, the mice in the obesity prevention and obesity treatment experimental groups were allowed to fast for 12 hours, tissues were separated and stored, and serum was separated. As a result of analyzing serum lipid indices, it was confirmed that free fatty acid (FIG. 2A) and triglyceride (FIG. 2B) decreased in the functional peptide administration group compared to the control.

Furthermore, as a result of observing adipose tissue by Hematoxylin and Eosin (H&E) staining and immunostaining, it was confirmed that the administration of the functional peptide reduced the area occupied by the adipocytes by 15%, and thus the functional peptide prevented lipid accumulation (FIG. 2C). The expression of uncoupling protein-1 (UCP-1), which is an activator of thermogenesis and energy homeostasis in adipose tissue, was also increased by 355% in the functional peptide administration group (FIG. 2D).

As a result of observing an adipose tissue obtained from the obesity treatment experimental group by immunostaining, it was confirmed that the anti-obesity effect of the functional peptide includes lipid accumulation and activation of energy metabolism by observing that the expression degree of UCP-1 was increased by 263%, similar to the results of the obesity prevention experimental group (FIG. 2E).

### 2-3. Mechanism analysis of anti-obesity effects of functional peptides

To differentiate 3T3-L1 adipocytes into adipocytes, 3T3-L1 adipocytes were cultured for total 8 days of 2 days in a primary differentiation medium (DMEM, 10% fetal bovine serum (FBS), 1% antibiotics, 0.5 mM 3-isobutyl-1-methylxanthine, 1 µM dexamethasone, and 10 µg/ml insulin) and 6 days in a secondary differentiation medium (DMEM, 10% FBS, 1% antibiotics, and 10 µg/ml insulin). In this case, each material (control: PBS, experimental group: 100 nM functional peptide) was added to the medium and used during the differentiation period.

As a result of the experiment, by confirming that all the levels of peroxisome proliferator-activated receptor gamma (PPAKγ), adiponectin, fatty acid-binding protein 4 (FABP4), CCAAT/enhancer-binding protein alpha (C/EBPα) and lipoprotein lipase (LPL) genes, which are indicators of adipose differentiation, were reduced in the functional peptide treatment groups, it could be seen that the functional peptide exhibits the anti-obesity effect by regulating adipose differentiation (FIG. 3A).

Furthermore, to confirm the appetite-regulating effect of the functional peptide in mHypoE-N41 hypothalamic cells, the hypothalamic cells were treated with PBS (control) and 100 nM mouse-derived functional peptide (experimental group) for 24 hours. As a result of observing the levels of agouti-related peptide (Agrp) and neuropeptide Y (NPY) genes, which are known to promote appetite, by collecting the cells after the experiment was completed, it was confirmed that the Agrp gene decreased by 50.2% and the NPY gene decreased by 48.8% compared to the control, and thus the functional peptide also has an appetite suppressing effect (FIG. 3B).

Similarly, as a result of observing the gene levels of appetite-related factors by treating hypothalamic cells with PBS (control) and 100 nM human-derived functional peptide (experimental group) for 24 hours, the Agrp gene decreased by 47.5% and the NPY gene decreased by 59.6% compared to the control, confirming the appetite suppressing effect of the human peptide (FIG. 3C).

Through the results of the present example, the anti-obesity effect of the functional peptide was finally verified.

### Example 3: Analysis of glucose metabolism-related efficacy of functional peptides

### 3-1. Breeding of type 2 diabetic mouse (db/db) model

To confirm the glucose metabolism-related effects of the functional peptide, type 2 diabetic mice (db/db) were used. A db/db model corresponding to type 2 diabetic mice is an animal model in which genetic mutations are induced in a leptin receptor responsible for dietary regulation, and is a representative model of diabetes in which obesity is induced because insulin signaling pathways and satiety cannot be regulated.

8-week-old male C57BLKS/6J-db/m+ (Hetero mutated) and C57BLKS/6J-db/db (Homo mutated) mice were distributed from Central Lab. Animal Inc., fed with a normal diet chow (ND) for 1 week, and subjected to an adaptation time. In this case, the temperature and humidity of the breeding space were maintained at all times at 18 to 24°C and at 50 to 60%, respectively, as breeding environments throughout the experimental period, and the mice were fed freely during both the adaptation period and the experimental period.

### 3-2. Analysis of glucose metabolism improvement efficacy of functional peptides

After the adaptation period, the mice were divided into a control and an experimental group, and each bred under the following conditions for 44 days. A normal diet was prepared as in Table 1 and provided.
db/m+ control: normal diet (ND) + intraperitoneal administration of PBS (n=10)
db/db control: normal diet (ND) + intraperitoneal administration of PBS (n=10)
db/db experimental group: normal diet (ND) + intraperitoneal administration of functional peptide (5 mg/kg) (n=10)

After the 44-day experimental period was completed, mice were allowed to fast for 14 hours, euthanized, and then dissected to collect tissues for additional analysis. Further, by collecting blood, serum was separated by centrifugation and analyzed.

Specifically, in order to evaluate the glucose metabolism improvement efficacy of functional peptides, the effect of improving glucose tolerance disorder caused by obesity and glucose tolerance disorder caused by gene mutation was confirmed through a glucose tolerance test.

First, at the end of the experiment, the obesity-prevention mouse model of Example 2-1 was allowed to fast for 12 hours, and a 20% glucose solution was injected intraperitoneally to observe changes in blood glucose value in blood. In this case, the corresponding material as in Example 3-1 was injected into each group 30 minutes before the injection of the glucose solution. As a result of confirming changes in blood glucose value, it could be seen that blood glucose levels decreased in the functional peptide administration group from 30 minutes after the glucose injection. Even in the results of quantifying the variations in total blood glucose (blood glucose value x time; GLUAUC), it was confirmed that the functional peptide has the effect of improving glucose metabolism by confirming the decrease in blood glucose value due to the functional peptide (FIG. 4).

The diabetic mouse model of Example 3-1 was allowed to fast for 12 hours, and a 20% glucose solution was injected intraperitoneally to observe changes in blood glucose value in blood. In this case, the corresponding material as in Example 3-1 was injected into each group 30 minutes before the injection of the glucose solution. As a result of confirming changes in blood glucose value, it could be seen that blood glucose levels significantly decreased in the functional peptide administration group compared to the db/db control after 60, 90, and 120 minutes of glucose injection. In addition, even in the results of quantifying the variations in total blood glucose (blood glucose level x time; GLUAUC), it was confirmed that the functional peptide has the effect of improving glucose metabolism by reducing the blood glucose value due to the functional peptide (FIG. 5B).

In order to observe changes and disorders in hormone secretion resulting from diabetes, tissues isolated from the diabetic mouse model of Example 3-1 were analyzed, and the weight of the pancreas, which plays a major role in hormone secretion, and the blood concentration of insulin hormone, which induces glucose absorption, were specifically tested.

As a result of the test, it was confirmed that the functional peptide positively affects the hormone secretion system by confirming that both the weight of the pancreas (FIG. 5C) and a value obtained by correcting the same with the body weight (FIG. 5D) increased in the functional peptide administration group. Furthermore, blood insulin secretion increased in the functional peptide administration group, so that it could be seen that hormone secretion was promoted by the functional peptide (FIG. 5E). In summary, it was finally verified that the functional peptide has a positive effect on metabolism.

## Claims

1. A peptide comprising an amino acid sequence represented by SEQ ID NO: 1 or 2.

2. A pharmaceutical composition for use in preventing or treating obesity, comprising the peptide of claim 1 as an active ingredient.

3. The pharmaceutical composition of claim 2, wherein the composition suppresses appetite.

4. The pharmaceutical composition of claim 3, wherein the appetite is suppressed by suppressing an agouti-related protein (AgRP) or neuropeptide Y (NPY) peptide.

5. The pharmaceutical composition of claim 2, wherein the composition suppresses lipid accumulation and differentiation of adipocytes.

6. The pharmaceutical composition of claim 2, wherein the composition increases the expression of an uncoupling protein-1 (UCP-1) gene, which activates thermogenesis and energy homeostasis in adipose tissue.

7. A pharmaceutical composition for preventing or treating diabetes or diabetic complications, comprising the peptide of claim 1 as an active ingredient.

8. The pharmaceutical composition of claim 7, wherein the composition promotes insulin secretion.

9. The pharmaceutical composition of claim 7, wherein the composition improves glucose tolerance.

10. A method for treating obesity, the method comprising administering the composition of claim 2 to an individual in need of treatment.

11. A method for treating diabetes or diabetic complications, the method comprising administering the composition of claim 7 to an individual in need of treatment.
